# FASCICULE DE BREVET EUROPEEN

(11) **EP 3 866 865 B1**
(45) Date de publication et mention de la délivrance du brevet: **22.01.2025**
(21) Numéro de dépôt: 19806018.8
(22) Date de dépôt: 15.10.2019
(51) Int. Cl.: A61L 9/20, B01D 53/00, F24F 3/16

(54) **DISPOSITIF D'ÉPURATION D'AIR POUR GRANDS VOLUMES**
LUFTREINIGUNGSVORRICHTUNG FÜR GROSSE RÄUME
AIR PURIFICATION DEVICE FOR LARGE SPACES

(30) Priorité: 18.10.2018 FR 1859629
(43) Date de publication de la demande: 25.08.2021
(73) Titulaire: UV Germi, 19240 Saint-Viance (FR)
(72) Inventeur: VIALLE, Pierre Jean, 19130 OBJAT (FR); BORDAS, Patrick, 19130 OBJAT (FR); BORDAS, André, 19130 OBJAT (FR)
(74) Mandataire: Ipside
(86) Numéro de dépôt international: PCT/FR2019/052434
(87) Numéro de publication internationale: WO 2020/079359

(56) Documents cités:
- CN-U- 204 121 979
- CN-U- 204 121 979
- JP-A- H11 104 225
- JP-A- H11 104 225
- KR-A- 20180 088 561
- KR-A- 20180 088 561
- US-A1- 2009 098 014
- US-A1- 2009 098 014
- US-A1- 2009 098 014
- US-B2- 9 737 841

## Description

### DOMAINE DE L'INVENTION

La présente invention concerne un dispositif d'épuration d'air pour des grands volumes intérieurs tels qu'un hall de laboratoire, un hall industriel, des espaces publics ou autre.

Plus particulièrement l'invention concerne un dispositif d'épuration d'air dans lequel l'air est purifié prioritairement par un module de photocatalyse conçu pour travailler à haut débit, et dont le traitement de purification d'air peut être complété au besoin par un module de photolyse et/ou un module de filtration par charbon actif.

### ARRIÈRE-PLAN TECHNOLOGIQUE

Il est estimé que les personnes passent de 80% à 90% de leur temps dans des espaces intérieurs, que ce soit sur un lieu de travail ou de résidence, une école, un espace public ou un moyen de transport. Or, une mauvaise qualité d'air intérieur peut générer des conséquences non négligeables pour la santé allant de la simple gêne olfactive, des yeux et de la peu, jusqu'à l'apparition des allergies, des troubles respiratoires, de cancer, des intoxications aiguës, *etc.*

En effet, nous retrouvons nombreux polluants de l'air extérieur qui migrent dans les espaces intérieurs où ils s'accumulent graduellement. Les polluants peuvent être aussi apportés par les occupants d'un local, voire par les matériaux de construction dudit local. Les principaux polluants de l'air intérieur sont des composants chimiques comme les composés organiques volatils (COV), oxydes d'azote (NOx), monoxyde de carbone (CO), hydrocarbures aromatiques polycycliques (HAP), phtalates, des composants biologiques comme les virus, les bactéries, les moisissures, les allergènes d'acarien ou des allergènes animaux, et des particules et fibres comme l'amiante, des fibres minérales artificielles, et de particules inertes.

Pour améliorer la qualité de l'air intérieur, il est souvent conseillé d'aérer ces espaces. Néanmoins, nos bâtiments sont de nos jours conçus pour optimiser l'espace et la consommation énergétique au détriment de sa capacité de ventilation. De plus, la qualité d'air extérieur n'est toujours pas optimale, et peut dans certains cas contribuer à détériorer la qualité de l'air intérieur, notamment lorsque l'on souhaite respecter des normes d'hygiène spécifiques pour un procédé comme dans l'industrie agroalimentaire ou pharmaceutique.

Les purificateurs d'air ont été ainsi proposés pour renouveler l'air des intérieurs. L'une des technologies utilisée depuis de nombreuses années est la radiation UV-C avec une longueur d'onde comprise entre 240 et 280 nm présentant un effet germicide grâce à des réactions photochimiques sur l'ADN des microorganismes. Néanmoins, pour éliminer également des composés chimiques, il est souvent fait appel aux propriétés oxydantes des rayons UV, par exemple avec la création d'ozone ou au moyen d'un média catalytique activé par l'énergie des UV comme dans la photocatalyse.

Par exemple, la demande de brevet américain N° US 2010/0143205 A1 propose un système purificateur d'air dit haut débit combinant les technologies de filtration et de rayons UV germicides et oxydatives. Le dispositif comporte une enceinte avec une entrée couplée à un préfiltre, et un ventilateur axial disposé auprès de l'entrée pour aspirer et souffler de l'air vers une chambre de réaction UV. Ladite chambre UV émet à 254 nm pour un effet germicide et à 185 nm pour la création d'ozone. L'air est soufflé à travers la chambre UV en créant une spirale entourant une lampe UV s'étendant longitudinalement dans l'axe du flux de l'air. L'air est ensuite envoyé vers une cartouche catalytique transformant l'ozone de la chambre en oxygène et purifiant l'air avant sa sortie. Ce dispositif utilise la création d'ozone pour améliorer le pouvoir purificateur de la chambre UV. Néanmoins ce type de dispositif possède l'inconvénient de présenter des fuites d'ozone et d'avoir une haute consommation énergétique.

En effet, l'un de problèmes majeurs avec les dispositifs d'épuration d'air réside dans le fait qu'ils restent efficients pendant une période initiale, puis leur efficacité commence à diminuer, et dans certains cas ils deviennent nuisibles pour la santé lorsqu'ils produisent des molécules indésirables comme sous-produits de réactions.

La demande de brevet français N° FR 2 920 670 A1 propose d'améliorer la consommation énergétique des systèmes d'épuration UV en employant les technologies de filtration, de photolyse et de photocatalyse dans un dispositif d'épuration. Ce dispositif comporte une enceinte sensiblement cylindrique disposée verticalement et comportant des grilles d'entrée d'air frontales dans la partie basse de l'enceinte, des ventilateurs au-dessous des entrées d'air aspirant et soufflant l'air vers le haut de l'enceinte à travers une chambre de photocatalyse et de photolyse. La photocatalyse et la photolyse sont réalisées simultanément au moyen d'une source de lumière UV-C entourée d'une cartouche de fibre de quartz imprégnée de dioxyde de titane s'étendant le long du réacteur, et au travers duquel le flux d'air passe vers une sortie supérieure d'air. Cette conception de dispositif reste néanmoins adaptée uniquement pour l'épuration de petits débits d'air, et une faille dans le système d'éclairage compromet toute l'efficience du dispositif. D'autre part, les utilisateurs ne sont pas en mesure de détecter un besoin de maintenance ou de réparation du dispositif. La demande de brevet US 2009/098014 A1 donne également un exemple d'un dispositif purificateur d'air combinant les technologies de photolyse et de photocatalyse. Ce purificateur n'est pas adapté aux grands débits d'air. KR20130041763A décrit aussi un exemple d'un purificateur d'air comprenant un cylindre photocatalytique.

### EXPOSÉ DE L'INVENTION

Un premier but de la présente invention est de proposer un dispositif d'épuration d'air adapté aux grands volumes d'air et dont l'utilisation et la configuration de ses composants soient flexibles.

Un autre but de l'invention, est de proposer un dispositif épurateur d'air dont la maintenance et la réparation soient aisées.

À cette fin, l'invention propose un dispositif d'épuration d'air selon la présente revendication 1.

La conception du module de photocatalyse permet d'optimiser le temps de contact avec le média photocatalytique et répartir les aires de contact dudit média avec l'air à purifier. En effet, lorsque l'air rentre au réacteur suivant une direction transversale vis-à-vis des parois des cylindres verticaux, l'air se distribue au sein du module de de photocatalyse sans turbulence excessive lorsqu'il est forcé à passer au travers de parois des cylindres photocatalytiques vers son intérieur.

Dans un mode de réalisation, le traitement d'air par photocatalyse est complété par un traitement par photolyse et le dispositif comporte un module de photolyse installé dans une section de l'enceinte séparée du module de photocatalyse, et comportant au moins une source lumineuse UV configurée pour travailler simultanément ou indépendamment du module de photocatalyse.

Le dispositif de l'invention est ainsi avantageusement conçu pour séparer dans le réacteur les étages de purification par photolyse et par photocatalyse, et utiliser ces deux technologies indépendamment l'une de l'autre.

Le dispositif est configuré avec au moins deux vitesses paramétrables de fonctionnement du ventilateur, une première vitesse déterminant un débit d'épuration d'air de l'ordre de 300 à 800 m³/h, et une deuxième vitesse déterminant un débit d'épuration d'air de l'ordre de 600 à 1600 m³/h.

Dans un mode de réalisation dans lequel le dispositif comporte un module de photolyse, la première vitesse du ventilateur détermine l'activation du module de photocatalyse, et la deuxième vitesse détermine l'activation simultanée du module de photocatalyse et du module de photolyse.

Le dispositif de l'invention peut comporter également toutes ou partie des caractéristiques suivantes considérées isolément ou en toute combinaison techniquement opérable.
- Deux entrées latérales d'air disposées face à face dans une partie basse de l'enceinte, les cellules de filtration desdites entrées comportant un capteur de pression et un filtre à particules en suspension PM1 démontable depuis l'extérieur de l'enceinte.
- La sortie d'air étant munie d'une cellule de filtration démontable depuis l'extérieur de l'enceinte et comportant un capteur de pression, un filtre à charbon et/ou un filtre de sortie de particules en suspension PM1, ladite sortie étant disposée dans une partie supérieure de l'enceinte dans un plan incliné et dans une zone irradiée par le module de photolyse.
- Un module de traitement par charbon actif comportant une cellule de filtration présentant un média chargé en charbon actif, ladite cellule de filtration étant installée sur des glissières dans un plan horizontal et avec un joint d'étanchéité avec les parois du réacteur.
- La sortie d'air comporte une grille comportant une première série d'ailettes parallèles et mobiles en rotation sur un axe vertical, et une deuxième série d'ailettes parallèles et mobiles en rotation dans un axe horizontal, un positionnement desdites ailettes permettant d'orienter un jet d'air purifié à la sortie du dispositif.
- L'enceinte comporte un corps vertical de section sensiblement carré ou rectangulaire, et l'intérieur de l'enceinte détermine un réacteur dans lequel sont installés : le module de photocatalyse dans une partie basse du réacteur et face aux entrées latérales d'air ; le ventilateur dans une partie intermédiaire haute du réacteur aspirant l'air au travers du module de photocatalyse et le soufflant vers le haut du réacteur et la sortie d'air ; et le module de photolyse et/ou le module de filtration par charbon actif dans une partie supérieure du réacteur entre la sortie d'air et le ventilateur.
- Le module de photocatalyse comporte cinq cylindres photocatalytiques disposés en quinconce à l'intérieur du réacteur, une paire de cylindres étant située face à chaque entrée latérale d'air et un cylindre au centre axial du réacteur, et chaque cylindre photocatalytique comporte une grille garnie de fibres de quartz imprégnées de dioxyde de titane déterminant une paroi du cylindre à l'intérieur duquel s'étend longitudinalement une lampe UV.
- Le module de photolyse comporte comme source lumineuse UV au moins quatre lampes UV disposées dans un plan horizontal, et espacées entre elles pour déterminer des passages de flux d'air entre lesdites lampes UV.
- Une plaque ajourée positionnée au-dessus du module de photolyse, dans laquelle des orifices sur ladite plaque déterminent des passages d'air et des rayonnements UV vers la sortie d'air. Ce mode de réalisation permet d'augmenter la durée de vie des filtres de la sortie d'air.
- Le module de photocatalyse et le module de photolyse comportent chacun au moins un capteur de fonctionnement configuré pour émettre un signal d'alerte en cas de dysfonctionnement d'une source lumineuse UV et/ou d'un ballast de lampe.
- Une unité centrale recueillant des données d'un signal fourni par un ou plusieurs capteurs du dispositif pour l'envoyer à une application de communication intégrée soit dans un téléphone mobile, soit dans une base de téléassistance, soit dans un serveur au travers d'un réseau LPWAN (Low Power Wide Area Network).
- L'application renseigne un état de fonctionnement du dispositif, et émet des alertes en cas d'un disfonctionnement et/ou de besoin de maintenance dudit dispositif.

### PRÉSENTATION DE FIGURES

D'autres avantages, buts et caractéristiques particulières de la présente invention ressortiront de la description qui va suivre, faite dans un but explicatif et nullement limitatif, en regard des dessins annexés.
Figure 1. Une vue en perspective de l'intérieur d'un mode de réalisation d'un dispositif d'épuration d'air selon l'invention.
Figure 2. Une vue en perspective de l'intérieur d'un mode de réalisation d'un dispositif d'épuration d'air selon l'invention.
Figure 3. Une vue en perspective de l'extérieur d'un dispositif d'épuration d'air selon l'invention.

### DESCRIPTION DÉTAILLÉE DE MODES DE RÉALISATION DE L'INVENTION

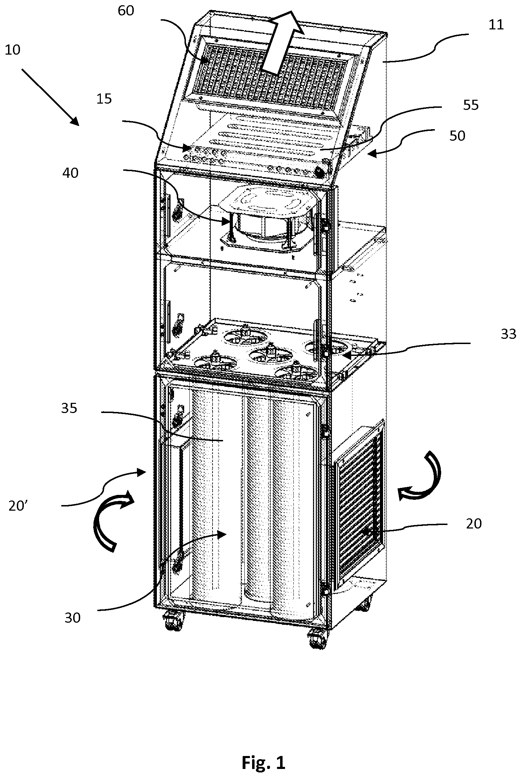

La figure 1 montre une représentation d'un exemple de dispositif purificateur d'air selon l'invention. Le dispositif 10 de l'invention combine trois technologies d'épuration d'air : la filtration, la photocatalyse et la photolyse. Le dispositif 10 comporte une enceinte 11 verticale creuse de section sensiblement rectangulaire présentant deux entrées 20, 20' latérales d'air disposées face à face et aspirant l'air depuis une partie basse de l'enceinte. L'intérieur de l'enceinte détermine un réacteur dans lequel l'air est purifié par un module de photocatalyse 30 et un module de photolyse 50 lorsqu'il suit un flux vers une sortie 60 située dans une partie supérieure de l'enceinte 11 où l'air purifié est expulsé du dispositif 10.

Le dispositif est illustré intégrant le module de photolyse, toutefois le dispositif peut comporter uniquement le module de photocatalyse conçu pour traiter efficacement un grand débit d'air. Suivant une application souhaitée, le dispositif comporte des modules complémentaires de purification tel que ledit module de photolyse et/ou un module de filtration par charbon actif à l'intérieur de l'enceinte.

Les entrées 20, 20' d'air et la sortie 60 d'air sont réalisées par exemple sous la forme des grilles, et comportent respectivement une cellule de filtration. Dans un mode préféré de réalisation, chaque cellule de filtration comporte un capteur de pression configuré pour détecter un niveau critique de perte de charge d'un filtre de ladite cellule, par exemple au moyen d'un différentiel de pression seuil détecté entre l'extérieur et l'intérieur du filtre. Avantageusement, les cellules de filtration sont remplaçables à partir de l'extérieur du dispositif d'épuration d'air.

L'épuration d'air commence donc par une filtration de particules en suspension aux entrées 20, 20' d'air de l'enceinte. Les filtres à particules en suspension sont de préférence des filtres PM1 avec une efficacité de filtration de 60 %. Les filtres PM1 permettent de retenir les particules d'un diamètre égal ou inférieur à 1 µm, les polluants de l'air considérés comme le plus dangereux pour la santé humaine en raison de leur petite taille capable de pénétrer le corps humain à travers le système respiratoire. Dans les particules en suspension PM1, nous retrouvons des allergènes, des bactéries, des virus, de la poussière en suspension, certains composés du smog et de la fumée de tabac. Bien évidemment, le filtre retient également les particules en suspension de taille supérieure à 1 µm comme la poussière medium et lourde, des spores de moisissure, du pollen, etc. Les cellules de filtration préviennent également l'encrassement du module de photocatalyse.

Avantageusement, deux entrées 20, 20' d'air sont disposées face à face pour repartir le passage d'air en deux, limitant ainsi la résistance au passage de l'air dans le réacteur, ainsi que de diviser la perte de charge par un facteur de 4 dans les cellules de filtration. De plus, cela permet au dispositif de fonctionner avec des débits importants en utilisant un ventilateur de puissance modérée.

L'air filtré rentre ainsi par les entrées 20, 20' latérales d'air dans la partie basse du réacteur où se situe également le module de photocatalyse 30. Dans le mode de réalisation illustré, le module de photocatalyse 30 comporte cinq cylindres photo catalytiques 35, chaque cylindre comportant une source lumineuse UV en son intérieur.

Les cinq cylindres photocatalytiques 35 sont disposés verticaux en quinconce à l'intérieur du réacteur, avec une paire de cylindres étant située face à chaque entrée latérale d'air et un cylindre au centre axial du réacteur. En outre, chaque cylindre photocatalytique comporte une grille garnie de fibres perméables à l'air et imprégnées d'un média photocatalytique. La grille avec le média photocatalytique déterminent une paroi du cylindre à l'intérieur duquel s'étend longitudinalement une lampe à vapeur de mercure. Par exemple, les parois sont définies par une grille métallique pourvue de fibres de quartz imprégnées de dioxyde de titane comme média photocatalytique.

Le module de photocatalyse permet d'épurer l'air grâce à l'oxydation de polluants au contact du média catalytique activé par l'énergie des photons émis par la source lumineuse UV. Les germes au contact du média photocatalytique sont complètement détruits ainsi que les toxines qu'ils libèrent lors de leur mort. La photocatalyse est très avantageuse aussi en ce qu'elle permet d'agir contre des polluants chimiques comme les composés organiques volatiles COV (acétaldéhyde, acétone, heptane, toluène..), les hydrocarbures aromatiques polycycliques, ainsi que des odeurs et d'autres molécules indésirables.

Avantageusement les cylindres photocatalytiques sont installés pour pouvoir être remplacés de manière unitaire et fonctionner indépendamment l'un de l'autre. Dans le circuit d'alimentation électrique de chaque cylindre se trouve un détecteur de fonctionnement de la lampe et du ballast de ladite lampe. Il est ainsi possible d'une part de maintenir le dispositif en activité lorsqu'un de ses cylindres présente un problème de fonctionnement, et d'autre part de prévenir les utilisateurs du dispositif de la présence d'une panne dans le module de photocatalyse.

Un ventilateur 40 d'air situé au-dessus du module de photocatalyse permet de générer le flux d'air à l'intérieur du réacteur d'épuration. L'air est aspiré par l'action du ventilateur au travers des entrées 20, 20' et rentre dans l'enceinte au niveau du module de photocatalyse 30 suivant un axe sensiblement transversal aux parois des cylindres photocatalytiques, pour être ensuite aspiré à l'intérieur des cylindres et ressortir au niveau de sections transversales supérieures desdits cylindres photocatalytiques. A la sortie du module de photocatalyse l'air est soufflé vers la sortie d'air, en passant par le module de photolyse 50 lorsque ce dernier est intégré au dispositif. L'air est donc d'abord aspiré à l'intérieur du réacteur et se répartit dans le module de photocatalyse sans turbulence excessive, ce qui permet d'améliorer le temps de contact avec le média photocatalytique, et de mieux répartir les aires de contact de l'air avec ledit média photocatalytique.

Dans le mode de réalisation illustré, le ventilateur est un ventilateur de type centrifuge positionné sur une plateforme comportant un orifice au travers duquel l'air est aspiré puis ressort à 90 ° pour être brassé autour du ventilateur lorsqu'il remonte vers le module de photolyse et la sortie 60.

D'autre part, le ventilateur 40 est avantageusement situé dans une partie intermédiaire haute du réacteur, relativement proche de la sortie d'air, ce qui permet de souffler l'air avec suffisamment de force pour l'expulser comme un courant d'air dirigé, tout en limitant la puissance nécessaire du ventilateur, ainsi que la nuisance sonore du dispositif. Par exemple, lorsque le dispositif de purification d'air est positionné avec la sortie 60 d'air orientée contre une paroi d'un local, un mouvement d'air est généré dans ledit local par le courant d'air qui remonte cette paroi, puis suit le plafond jusqu'à redescendre par une autre paroi, créant ainsi un mouvement d'air au sein du local sans déranger les zones du local occupées par des personnes. Dans un mode de réalisation, le jet d'air de sortie est orienté grâce à une première série d'ailettes verticales mobiles en rotation vers la gauche et vers la droite, et une deuxième série d'ailettes horizontales, mobiles vers le haut et vers le bas.

Dans le mode de réalisation illustré (fig. 1), le dispositif épurateur d'air de l'invention comporte également une barrière d'étanchéité 33 au-dessus des cylindres photocatalytiques permettant de contrôler les sorties d'air du module de photocatalyse 30 vers une partie supérieure du réacteur. Par exemple, lesdites sorties d'air correspondent à des ouvertures situées uniquement au niveau des sections transversales supérieures des cylindres photocatalytiques. L'air est ainsi forcé à passer vers l'intérieur des cartouches photocatalytiques, puis il ressort par les sections supérieures des cylindres avant d'être aspiré par le ventilateur et propulsé vers le haut de l'enceinte.

Un module de photolyse 50 est disposé dans une partie supérieure du réacteur d'épuration, au-dessus du ventilateur 40 d'air. Le module de photolyse permet d'augmenter l'efficience d'épuration du dispositif en agissant sur les polluants qui ont échappé au traitement par photocatalyse. Dans un mode de réalisation, le module de photolyse 50 comporte cinq lampes UV à vapeur de mercure. Les lampes sont disposées dans une position horizontale, perpendiculaire au flux d'air, et sont espacées entre elles de manière à déterminer des passages d'air entre lesdites lampes UV vers la sortie d'air. Cette disposition des lampes permet d'augmenter les angles d'exposition des particules de pollution aux rayonnements UV, et donc de favoriser leur action germicide.

Le module de photolyse 50 est positionné avantageusement en dessous de la sortie 60 d'air comportant une cellule de filtration munie d'un filtre à charbon et/ou un filtre à particules, les filtres de la sortie d'air étant ainsi exposés aux rayonnements UV. Le développement de moisissure et d'autres microorganismes retenus à la cellule de filtration de sortie est donc empêché grâce à l'exposition de la cellule de filtration aux rayonnements UV.

Dans un mode de réalisation préféré, le dispositif comporte une plaque ajourée comportant des orifices déterminant des passages d'air et de rayonnements UV vers une partie supérieure du réacteur et vers la sortie d'air. Ce mode de réalisation est très avantageux en ce que ladite plaque va permettre de limiter la quantité des rayonnements UV auxquels sera exposée la cellule de filtration de la sortie 60. Ceci permet d'éviter le vieillissement précoce du filtre à charbon et/ou du filtre à particules en suspension, tout en conservant un effet germicide sur lesdits filtres.

La plaque ajourée est par exemple une plaque horizontale comportant des orifices tubulaires sensiblement superposés aux espaces entre les lampes UV du module de photocatalyse, les orifices laissent donc passer la lumière indirecte et la plaque forme un écran contre les rayons qui seraient diffusés directement vers les filtres de la sortie. Les dimensions des orifices sont donc adaptées aux espacements entre les lampes, ceci afin que les portions pleines de la plaque soient au-dessus des lampes. La plaque peut être réalisée en inox et elle n'est pas en contact direct avec les lampes.

Le dispositif de l'invention est particulier en ce que le module de photocatalyse est séparé et indépendant du module de photolyse. Cette conception permet d'utiliser les conditions opératoires optimales pour chaque module. Par exemple, d'utiliser des lampes de plus basse consommation énergétique pour le module de photolyse, et des lampes plus puissantes pour le module de photocatalyse. A titre d'exemple, les lampes utilisées sont des lampes UV-C de basse pression émettant à une longueur d'onde de 254 nm, .

La séparation fonctionnelle du module de photocatalyse et du module de photolyse permet également de configurer son utilisation en fonction du volume d'air à purifier. En effet, le dispositif de l'invention comporte au moins deux débits de purification d'air déterminés en outre au moyen d'une vitesse du ventilateur.

Dans un mode de réalisation préféré, le dispositif fournit deux débits d'air, un premier débit de 600 m³ /h, et un deuxième débit de 1100 m³ /h. Le débit d'air souhaité est par exemple sélectionné au moyen d'un interrupteur à l'extérieur du dispositif.

Dans un mode de réalisation, la sélection de la deuxième vitesse d'air du ventilateur active également les lampes du module de photolyse.

Le dispositif de l'invention peut être ainsi avantageusement configuré pour actionner uniquement le module de photocatalyse lorsque le premier débit de 600 m³ /h est sélectionné, le module de photocatalyse étant suffisant pour épurer ledit débit d'air.

Lorsque le deuxième débit d'air est sélectionné, le dispositif active simultanément le module de photocatalyse et le module de photolyse permettant ensemble de conserver l'efficacité du dispositif à un débit de 1100 m³/h.

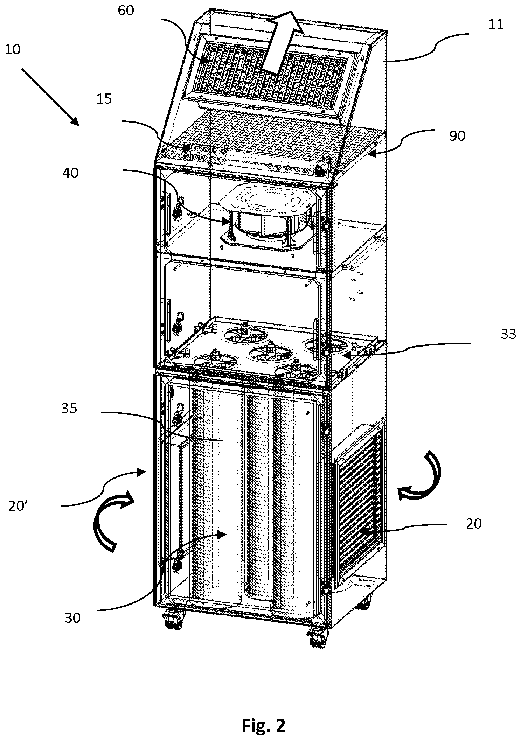

Dans un autre mode de réalisation du dispositif (Figure 2), le module de photolyse est remplacé ou complété par le module de traitement par charbon actif 90. Ce module comporte une cellule de filtration avec un filtre plan comportant un média chargé en charbon actif et un joint d'étanchéité entre un périmètre extérieur de ladite cellule de filtration et le réacteur. Le joint d'étanchéité assure donc le passage du flux d'air par le média de la cellule de filtration.

Avantageusement, le module de traitement par charbon actif est installé sur des glissières facilitant son installation et maintenance.

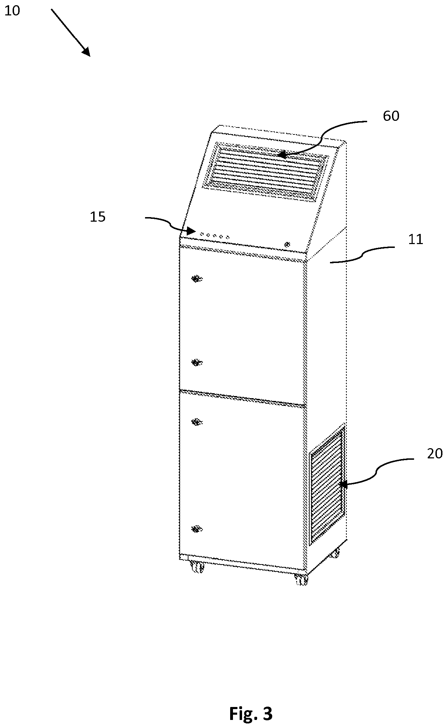

La figure 3 montre un exemple de réalisation du dispositif vu depuis l'extérieur. Le dispositif de l'invention comporte de préférence une interface comportant des leds 15 indicatrices du fonctionnement du dispositif. Par exemple :
- une led bleu témoin de mise sous tension ;
- une led verte témoin de la marche de la photocatalyse ;
- une led verte témoin de la marche de la photolyse ;
- une led rouge témoin pour défaut de marche ;
- une led rouge pour signaler le besoin de changement des filtres.

Dans le mode de réalisation illustré, le dispositif de l'invention permet de purifier au moins 1100 m³ /h avec une puissance électrique maximale de 650 W, branché sur une prise de 230 V, tout en ayant un niveau sonore inférieur à 50 dBA à une distance de 2m en champ libre.

Dans un mode de réalisation complémentaire, le dispositif d'épuration d'air comporte une unité centrale recueillant l'information du signal fourni par les différents capteurs du dispositif pour l'envoyer à une application de communication intégrée soit dans un téléphone mobile, soit dans une base de téléassistance, soit dans un serveur au travers d'un réseau LPWAN (Low Power Wide Area Network).

L'application de communication à distance permet de renseigner un utilisateur sur l'état de fonctionnement de l'appareil au moyen d'une interface logiciel et/ou par l'envoi de messages SMS à un numéro configuré. A titre d'exemple, l'application renseigne un état normal, ou un défaut de fonctionnement tel qu'une panne de lampes, de ballast et/ou des filtres. L'application de communication permet également de faire un suivi dans le temps de la durée de vie des composants du dispositif, et d'émettre des alertes de maintenance pour le changement d'un composant quand il atteint ou il approche sa fin de vie, par exemple pour le changement des lampes.

Dans un mode de réalisation, le dispositif communique avec l'application de communication au moyen d'une puce de communication ou une connexion ethernet.

Afin de faciliter la maintenance ou la réparation du dispositif, l'une des parois du corps de l'enceinte est configurée comme une ou plusieurs portes de maintenance s'ouvrant vers l'extérieur et donnant accès à l'intégralité des composants du réacteur du dispositif. Les dimensions en mètres du dispositif sont d'environ 1,90 x 0,5 x 0,6 et le dispositif est mis sur roulettes pour faciliter son déplacement. Le dispositif peut comporter également des couvercles pour protéger les cellules de filtration et l'intérieur du réacteur lorsque le dispositif n'est pas en fonctionnement.

L'invention propose ainsi un dispositif flexible dans son utilisation et dans la configuration de ses composants pour adapter ses étages de purification et sa consommation électrique en fonction d'un besoin de débit d'air purifié, et d'assurer une épuration d'air minimale lorsqu'un de ses composants tombe en panne ou ne fonctionne pas correctement.

Le dispositif de l'invention est particulièrement adapté à des usages professionnels pour maintenir ou assurer une qualité d'air intérieur nécessaire à des procédés industriels, ou pour améliorer les conditions de travail de personnes travaillant dans des industries dans lesquelles les procédés génèrent des polluants pouvant affecter la santé des employés.

## Revendications

1. Dispositif (10) d'épuration d'air comportant une enceinte (11) verticale avec des entrées (20, 20') latérales d'air munies d'une cellule de filtration, un réacteur d'épuration d'air comportant un module de photocatalyse (30) comportant des cylindres (35) photocatalytiques avec au moins une source lumineuse UV en son intérieur, un ventilateur (40) avec un moteur et une sortie (60) d'air purifié, , **caractérisé en ce que :**
- les cylindres (35) photocatalytiques sont disposés verticaux, suivant un plan vertical de l'enceinte et face aux entrées latérales d'air (20, 20'), et lesdits cylindres (35) comportent des parois habillées d'un média photocatalytique et présentant une perméabilité à l'air et
- le dispositif (10) comporte une barrière d'étanchéité (33) au-dessus du module de photocatalyse (30) comportant au moins une ouverture au niveau de la section transversale supérieure de chacun des cylindres photocatalytiques dudit module, et **en ce que**
- l'air rentre dans l'enceinte au niveau du module de photocatalyse (30) suivant un axe sensiblement transversal aux parois des cylindres photocatalytiques, pour être ensuite aspiré au travers des parois des cylindres à l'intérieur desdits cylindres (35), et ressortir au niveau de sections transversales supérieures desdits cylindres (35) photocatalytiques et par lesdites ouvertures de la barrière d'étanchéité déterminant des sorties d'air du module de photocatalyse vers le haut du réacteur.

2. Dispositif selon la revendication 1, dans lequel le réacteur d'épuration d'air comporte un module de photolyse (50) installé dans une section de l'enceinte séparée du module de photocatalyse, et comportant au moins une source lumineuse UV configurée pour travailler simultanément ou indépendamment du module de photocatalyse.

3. Dispositif selon l'une des revendications précédentes, comportant au moins deux vitesses paramétrables de fonctionnement du ventilateur (40), une première vitesse déterminant un débit d'épuration d'air de l'ordre de 300 à 800 m³/h, et une deuxième vitesse déterminant un débit d'épuration d'air de l'ordre de 600 à 1600 m³/h.

4. Dispositif selon la revendication 3 en combinaison avec la revendication 2, dans lequel le réacteur comporte un module de photolyse (50), et la première vitesse du ventilateur (40) détermine l'activation du module de photocatalyse (30), et la deuxième vitesse détermine l'activation simultanée du module de photocatalyse (30) et du module de photolyse (50).

5. Dispositif selon l'une des revendications précédentes, dans lequel le dispositif (10) comporte deux entrées (20, 20') latérales d'air disposées face à face dans une partie basse de l'enceinte (11), et les cellules de filtration desdites entrées comportent un capteur de pression et un filtre à particules en suspension PM1 démontable depuis l'extérieur de l'enceinte.

6. Dispositif selon l'une des revendications précédentes, dans lequel la sortie (60) d'air est munie d'une cellule de filtration démontable depuis l'extérieur de l'enceinte et comportant un capteur de pression, un filtre à charbon et/ou un filtre de sortie de particules en suspension PM1, et ladite sortie (60) est disposée dans une partie supérieure de l'enceinte dans un plan incliné et dans une zone irradiée par le module de photolyse.

7. Dispositif selon l'une des revendications 1 à 6, dans lequel le dispositif comporte un module de traitement par charbon actif (90) comportant une cellule de filtration présentant un média chargé en charbon actif, ladite cellule de filtration étant installée sur des glissières dans un plan horizontal et avec un joint d'étanchéité à l'intérieur de l'enceinte.

8. Dispositif selon l'une des revendications précédentes, dans lequel la sortie d'air comporte une grille comportant une première série d'ailettes parallèles et mobiles en rotation sur un axe vertical, et une deuxième série d'ailettes parallèles et mobiles en rotation dans un axe horizontal, le positionnement desdites ailettes permettant d'orienter un jet d'air purifié à la sortie du dispositif.

9. Dispositif selon l'une des revendications précédentes, dans laquelle l'enceinte (11) comporte un corps vertical de section sensiblement carrée ou rectangulaire, et l'intérieur de l'enceinte détermine le réacteur d'épuration dans lequel sont installés :
- le module de photocatalyse (30) dans une partie basse du réacteur et face aux entrées (20, 20') latérales d'air ;
- le ventilateur (40) dans une partie intermédiaire haute du réacteur aspirant l'air au travers du module de photocatalyse (30) et le soufflant vers le haut du réacteur et la sortie d'air ;
- le module de photolyse (50) et/ou le module de filtration par charbon actif (90) dans une partie supérieure du réacteur entre la sortie (60) d'air et le ventilateur (40).

10. Dispositif selon l'une des revendications précédentes, dans lequel le module de photocatalyse (30) comporte cinq cylindres (35) photocatalytiques disposés en quinconce à l'intérieur du réacteur, une paire de cylindres (35) étant située face à chaque entrée latérale d'air et un cylindre (35) au centre axial du réacteur, et chaque cylindre photocatalytique comporte une grille garnie de fibres de quartz imprégnées de dioxyde de titane déterminant une paroi du cylindre à l'intérieur duquel s'étend longitudinalement une lampe UV.

11. Dispositif selon l'une des revendications 2 à 10, dans lequel le réacteur d'épuration d'air comporte ledit module de photolyse, et ledit module de photolyse (50) comporte comme source lumineuse UV au moins cinq lampes UV disposées dans un plan horizontal, et espacées entre elles pour déterminer des passages de flux d'air entre lesdites lampes UV.

12. Dispositif selon l'une des revendications 2 à 11, dans lequel le réacteur d'épuration d'air comporte ledit module de photolyse (50), et une plaque (55) ajourée positionnée au-dessus du module de photolyse, dans laquelle des orifices sur ladite plaque déterminent des passages d'air et des rayonnements UV vers la sortie d'air (60).

13. Dispositif selon l'une des revendications 2 à 12, dans lequel le réacteur d'épuration d'air comporte ledit module de photolyse, et dans lequel le module de photocatalyse (30) et/ou le module de photolyse (50) comportent chacun au moins un capteur de fonctionnement configuré pour émettre un signal d'alerte en cas de dysfonctionnement d'une source lumineuse UV et/ou d'un ballast de lampe.

14. Dispositif selon l'une des revendications précédentes, comportant une unité centrale recueillant des données d'un signal fourni par un ou plusieurs capteurs du dispositif pour l'envoyer à une application de communication intégrée soit dans un téléphone mobile, soit dans une base de téléassistance, soit dans un serveur au travers d'un réseau LPWAN (Low Power Wide Area Network), et dans lequel l'application renseigne un état de fonctionnement du dispositif (10), et émet des alertes en cas d'un dysfonctionnement et/ou de besoin de maintenance dudit dispositif.

## Patentansprüche

1. Luftreinigungsvorrichtung (10), die ein vertikales Gehäuse (11) mit seitlichen Lufteinlässen (20, 20'), die mit einer Filterzelle ausgestattet sind, einen Luftreinigungsreaktor, der ein Photokatalysemodul (30) umfasst, das photokatalytische Zylinder (35) mit mindestens einer UV-Lichtquelle in seinem Inneren umfasst, einen Lüfter (40) mit Motor und einen Auslass (60) für gereinigte Luft umfasst, **dadurch gekennzeichnet, dass**:
- die photokatalytischen Zylinder (35) vertikal entlang einer vertikalen Ebene des Gehäuses angeordnet und den seitlichen Lufteinlässen (20, 20') zugewandt sind, und die Zylinder (35) Wände umfassen, die mit einem photokatalytischen Medium überzogen sind und eine Luftdurchlässigkeit aufweisen; und
- die Vorrichtung (10) eine Dichtungsbarriere (33) über dem Photokatalysemodul (30) umfasst, die mindestens eine Öffnung auf Höhe des oberen Querschnitts jedes der photokatalytischen Zylinder des Moduls umfasst, und dadurch, dass
- die Luft auf Höhe des Photokatalysemoduls (30) entlang einer Achse, die im Wesentlichen quer zu den Wänden der photokatalytischen Zylinder verläuft, in das Gehäuse eintritt, um dann durch die Wände der Zylinder im Inneren der Zylinder (35) angesaugt zu werden und auf Höhe der oberen Querschnitte der photokatalytischen Zylinder (35) und durch die Öffnungen der Dichtungsbarriere, die die Luftauslässe des Photokatalysemoduls in Richtung der Oberseite des Reaktors festlegen, auszutreten.

2. Vorrichtung nach Anspruch 1, wobei der Luftreinigungsreaktor ein Photolysemodul (50) umfasst, das in einem von dem Photokatalysemodul getrennten Abschnitt des Gehäuses installiert ist und mindestens eine UV-Lichtquelle umfasst, die dazu konfiguriert ist, gleichzeitig mit dem Photokatalysemodul oder unabhängig von diesem zu arbeiten.

3. Vorrichtung nach einem der vorhergehenden Ansprüche, die mindestens zwei einstellbare Betriebsgeschwindigkeiten für den Lüfter (40) umfasst, wobei eine erste Geschwindigkeit eine Luftreinigungsrate in der Größenordnung von 300 bis 800 m³ /h festlegt und eine zweite Geschwindigkeit eine Luftreinigungsrate in der Größenordnung von etwa 600 bis 1600 m³ /h festlegt.

4. Vorrichtung nach Anspruch 3 in Kombination mit Anspruch 2, wobei der Reaktor ein Photolysemodul (50) umfasst und die erste Geschwindigkeit des Lüfters (40) die Aktivierung des Photokatalysemoduls (30) festlegt und die zweite Geschwindigkeit die gleichzeitige Aktivierung des Photokatalysemoduls (30) und des Photolysemoduls (50) festlegt.

5. Vorrichtung nach einem der vorhergehenden Ansprüche, wobei die Vorrichtung (10) zwei seitliche Lufteinlässe (20, 20') umfasst, die einander gegenüberliegend in einem unteren Abschnitt des Gehäuses (11) angeordnet sind, und die Filterzellen der Einlässe einen Drucksensor und einen PM1-Feinstaubfilter, der von außerhalb des Gehäuses demontiert werden kann, umfassen.

6. Vorrichtung nach einem der vorhergehenden Ansprüche, wobei der Luftauslass (60) mit einer Filterzelle ausgestattet ist, die von außerhalb des Gehäuses demontiert werden kann und einen Drucksensor, einen Kohlefilter und/oder einen Auslassfilter für PM1-Feinstaub umfasst, und der Auslass (60) in einem oberen Abschnitt des Gehäuses in einer geneigten Ebene und in einem vom Photolysemodul bestrahlten Bereich angeordnet ist.

7. Vorrichtung nach einem der Ansprüche 1 bis 6, wobei die Vorrichtung ein Aktivkohle-Behandlungsmodul (90) umfasst, das eine Filterzelle umfasst, die ein mit Aktivkohle beladenes Medium aufweist, wobei die Filterzelle auf Schlitten in einer horizontalen Ebene und mit einer Dichtung im Inneren des Gehäuses installiert ist.

8. Vorrichtung nach einem der vorhergehenden Ansprüche, wobei der Luftauslass ein Gitter umfasst, das eine erste Reihe paralleler Rippen, die auf einer vertikalen Achse drehbeweglich sind, und eine zweite Reihe paralleler Rippen umfasst, die auf einer horizontalen Achse drehbeweglich sind, wobei die Positionierung der Rippen es ermöglicht, einen Strahl gereinigter Luft auf den Auslass der Vorrichtung auszurichten.

9. Vorrichtung nach einem der vorhergehenden Ansprüche, wobei das Gehäuse (11) einen vertikalen Körper mit einem im Wesentlichen quadratischen oder rechteckigen Querschnitt umfasst, und das Innere des Gehäuses den Reinigungsreaktor festlegt, in dem Folgendes installiert ist:
- das Photokatalysemodul (30) in einem unteren Abschnitt des Reaktors und den seitlichen Lufteinlässen (20, 20') zugewandt;
- der Lüfter (40) in einem oberen Zwischenabschnitt des Reaktors, der Luft durch das Photokatalysemodul (30) ansaugt und diese in Richtung der Oberseite des Reaktors und des Luftauslasses bläst;
- das Photolysemodul (50) und/oder das Aktivkohle-Filtrationsmodul (90) in einem oberen Abschnitt des Reaktors zwischen dem Luftauslass (60) und dem Lüfter (40).

10. Vorrichtung nach einem der vorhergehenden Ansprüche, wobei das Photokatalysemodul (30) fünf photokatalytische Zylinder (35) umfasst, die versetzt im Inneren des Reaktors angeordnet sind, wobei sich jeweils ein Paar Zylinder (35) gegenüber jedem seitlichen Lufteinlass und ein Zylinder (35) in der axialen Mitte des Reaktors befindet, und jeder photokatalytische Zylinder ein Gitter umfasst, das mit mit Titandioxid imprägnierten Quarzfasern ausgekleidet ist und eine Wand des Zylinders im Inneren festlegt, von der sich eine UV-Lampe in Längsrichtung erstreckt.

11. Vorrichtung nach einem der Ansprüche 2 bis 10, wobei der Luftreinigungsreaktor das Photolysemodul umfasst und das Photolysemodul (50) als UV-Lichtquelle mindestens fünf UV-Lampen umfasst, die in einer horizontalen Ebene angeordnet und voneinander beabstandet sind, um Luftströmungskanäle zwischen den UV-Lampen festzulegen.

12. Vorrichtung nach einem der Ansprüche 2 bis 11, wobei der Luftreinigungsreaktor das Photolysemodul (50) und eine über dem Photolysemodul positionierte Lochplatte (55) umfasst, in der Öffnungen auf der Platte die Luftkanäle und UV-Strahlung in Richtung des Luftauslasses (60) festlegen.

13. Vorrichtung nach einem der Ansprüche 2 bis 12, wobei der Luftreinigungsreaktor das Photolysemodul umfasst, und wobei das Photokatalysemodul (30) und/oder das Photolysemodul (50) jeweils mindestens einen Betriebssensor umfassen, der dazu konfiguriert ist, im Falle einer Fehlfunktion einer UV-Lichtquelle und/oder eines Lampenvorschaltgeräts ein Warnsignal auszusenden.

14. Vorrichtung nach einem der vorhergehenden Ansprüche, die eine Zentraleinheit umfasst, die Daten aus einem Signal sammelt, das von einem oder mehreren Sensoren der Vorrichtung geliefert wird, um sie über ein LPWAN (Low Power Wide Area Network) an eine Kommunikationsanwendung zu senden, die entweder in einem Mobiltelefon oder in einer Fernunterstützung-Basisstation oder in einem Server integriert ist, und wobei die Anwendung über einen Betriebsstatus der Vorrichtung (10) informiert und im Falle einer Fehlfunktion und/oder eines Wartungsbedarfs Warnungen der Vorrichtung Alarme aussendet.

## Claims

1. An air purification device (10) including a vertical enclosure (11) with lateral air inlets (20, 20') provided with a filtration cell, an air purification reactor including a photocatalysis module (30) including photocatalytic cylinders (35) with at least one UV light source inside, a fan (40) with a motor and a purified air outlet (60), **characterized in that:**
- the photocatalytic cylinders (35) are disposed vertically, along a vertical plane of the enclosure and facing the lateral air inlets (20, 20'), and said cylinders (35) include walls covered with a photocatalytic medium and having an air permeability; and
- the device (10) includes a sealing barrier (33) above the photocatalysis module (30) including at least one opening at the upper cross-section of each of the photocatalytic cylinders of said module, and **in that**
- the air enters the enclosure at the photocatalysis module (30) along an axis substantially transverse to the walls of the photocatalytic cylinders, to then be sucked through the walls of the cylinders inside said cylinders (35), and exit at the upper cross-sections of said photocatalytic cylinders (35) and through said openings of the sealing barrier determining air outlets from the photocatalysis module to the top of the reactor.

2. The device according to claim 1, wherein the air purification reactor includes a photolysis module (50) installed in a section of the enclosure separate from the photocatalysis module, and including at least one UV light source configured to work simultaneously or independently of the photocatalysis module.

3. The device according to one of the preceding claims, including at least two configurable operating speeds of the fan (40), a first speed determining an air purification flow rate in the range of 300 to 800 m³/h, and a second speed determining an air purification flow rate in the range of 600 to 1600 m³/h.

4. The device according to claim 3 in combination with claim 2, wherein the reactor includes a photolysis module (50), and the first speed of the fan (40) determines the activation of the photocatalysis module (30), and the second speed determines the simultaneous activation of the photocatalysis module (30) and the photolysis module (50).

5. The device according to one of the preceding claims, wherein the device (10) includes two lateral air inlets (20, 20') disposed face to face in a lower portion of the enclosure (11), and the filtration cells of said inlets include a pressure sensor and a suspended particle filter PM1 removable from outside the enclosure.

6. The device according to one of the preceding claims, wherein the air outlet (60) is provided with a filtration cell removable from the outside of the enclosure and including a pressure sensor, a carbon filter and/or a suspended particle outlet filter PM1, and said outlet (60) is disposed in an upper portion of the enclosure in an inclined plane and in an area irradiated by the photolysis module.

7. The device according to one of claims 1 to 6, wherein the device includes an activated carbon treatment module (90) including a filtration cell having a media loaded with activated carbon, said filtration cell being installed on slides in a horizontal plane and with a seal inside the enclosure.

8. The device according to one of the preceding claims, wherein the air outlet includes a grid including a first series of fins which are parallel and movable in rotation on a vertical axis, and a second series which are parallel and movable in rotation on a horizontal axis, the positioning of said fins allowing directing a jet of purified air at the outlet of the device.

9. The device according to one of the preceding claims, wherein the enclosure (11) includes a vertical body of substantially square or rectangular section, and the interior of the enclosure determines the purification reactor in which are installed:
- the photocatalysis module (30) in a lower portion of the reactor and facing the lateral air inlets (20, 20');
- the fan (40) in an upper intermediate portion of the reactor sucking air through the photocatalysis module (30) and blowing it towards the top of the reactor and the air outlet;
- the photolysis module (50) and/or the activated carbon filtration module (90) in an upper portion of the reactor between the air outlet (60) and the fan (40).

10. The device according to one of the preceding claims, wherein the photocatalysis module (30) includes five photocatalytic cylinders (35) disposed in a staggered arrangement inside the reactor, a pair of cylinders (35) being located facing each lateral air inlet and a cylinder (35) at the axial center of the reactor, and each photocatalytic cylinder includes a grid filled with quartz fibers impregnated with titanium dioxide determining a wall of the cylinder inside which a UV lamp extends longitudinally.

11. The device according to one of claims 2 to 10, wherein the air purification reactor includes said photolysis module, and said photolysis module (50) includes as a UV light source at least five UV lamps disposed in a horizontal plane, and spaced apart to determine air flow passages between said UV lamps.

12. The device according to one of claims 2 to 11, wherein the air purification reactor includes said photolysis module (50), and a perforated plate (55) positioned above the photolysis module, wherein orifices on said plate determine air passages and UV radiation towards the air outlet (60).

13. The device according to one of claims 2 to 12, wherein the air purification reactor includes said photolysis module, and wherein the photocatalysis module (30) and/or the photolysis module (50) each include at least one operating sensor configured to emit an alert signal in the case of a malfunction of a UV light source and/or a lamp ballast.

14. The device according to one of the preceding claims, including a central unit collecting data from a signal provided by one or more sensors of the device to send it to a communication application integrated either in a mobile telephone, or in a remote assistance base, or in a server via an LPWAN (Low Power Wide Area Network), and wherein the application provides information on an operating status of the device (10), and issues alerts in the case of a malfunction and/or need for maintenance of said device.
